# EUROPEAN PATENT APPLICATION

(11) **EP 4 721 776 A1**
(43) Date of publication of application: **08.04.2026**
(21) Application number: 24823492.4
(22) Date of filing: 17.06.2024
(51) Int. Cl.: A61M 1/02

(54) **FILTER DEVICE**

(30) Priority: 16.06.2023 JP 2023099348
(71) Applicant: Terumo Kabushiki Kaisha, Tokyo 151-0072 (JP)
(72) Inventor: IIDA, Naoki, Fujinomiya-shi, Shizuoka 418-0004 (JP)
(74) Representative: TBK
(86) International application number: PCT/JP2024/021794
(87) International publication number: WO 2024/257879

(57) **Abstract**

A filter device (10) according to the present invention is to be mounted on a centrifugal separator (50) and includes: a first space (18) into which blood flows; a second space (20) from which the blood flows out; a partition wall (22) separating the first space and the second space; a communication port (28) establishing communication between the first space and the second space; and a white-blood-cell removal filter (24) disposed in the second space. The first space and the second space are arranged in a first direction (D1) intersecting a centrifugal direction (DC), and the inlet port (14a) and the communication port are located between a center line (C12) along the first direction and a rotation center (LA) of the centrifugal separator.

## Description

### Technical Field

The present invention relates to a filter device.

### Background Art

JP 5223006 B2 describes a blood bag system and a centrifugal transfer device (centrifugal separator). The blood bag system accommodates blood. The centrifugal separator centrifuges the blood accommodated in the blood bag system. The centrifuged blood is used for blood transfusion.

### Citation List

### Patent Literature

Patent Literature 1: JP 5223006 B2

### Summary of Invention

It is preferable that the blood to be transfused does not contain white blood cells. Recently, a filter device capable of more suitably removing white blood cells is desired.

It is therefore an object of the present invention to solve the above-described problem.
(1) One aspect of the present invention provides a filter device to be mounted on a centrifugal separator, the filter device including: a first space which is formed in a housing and into which blood flows through an inlet port; a second space which is formed in the housing and from which the blood flows out through an outlet port; a partition wall that separates the first space and the second space; a communication port that establishes communication between the first space and the second space; and a white-blood-cell removal filter that is disposed in the second space and that removes a white blood cell contained in the blood, wherein the first space and the second space are arranged in a first direction intersecting a centrifugal direction in which a centrifugal force is applied, and the inlet port and the communication port are located between a center line of the housing along the first direction and a rotation center of the centrifugal separator.
   With this configuration, white blood cells can be suitably removed from blood.
(2) In the filter device according to (1), the outlet port may be located in the centrifugal direction relative to the center line.
   With this configuration, the blood can be smoothly taken out from the outlet port only by operating the centrifugal separator.
(3) In the filter device according to (1) or (2), the inlet port may be located on one side of the first space in the first direction, and the communication port may be located on another side of the first space in the first direction.
   This configuration makes it possible to prevent blood from which white blood cells have not been removed by centrifugation from reaching the second space.
(4) In the filter device according to any one of (1) to (3), the first space may include a curved wall, and the curved wall may be located in the centrifugal direction relative to the center line and located on one side of the first space.
   This configuration makes it possible to prevent backflow of blood toward the inlet port.
(5) In the filter device according to any one of (1) to (4), the communication port may be located on one side of the second space in the first direction, and the outlet port may be located on another side of the second space in the first direction.
   This configuration makes it possible to more reliably remove white blood cells by the white-blood-cell removal filter.
(6) In the filter device according to any one of (1) to (5), the first space may be provided with a meandering flow channel for allowing the blood flowing in through the inlet port to meander.

This configuration makes it possible to prevent blood from which white blood cells have not been removed by centrifugation from reaching the second space.

According to the present invention, it is possible to suitably remove white blood cells.

### Brief Description of Drawings

Fig. 1 is a perspective view illustrating a centrifugal separator to which a filter device according to an embodiment is mounted.
Fig. 2 is a perspective view illustrating the filter device.
Fig. 3 is a plan view illustrating an internal structure of the filter device.
Fig. 4 is a plan view illustrating an internal structure of a filter device according to a first modification.
Fig. 5 is a plan view illustrating an internal structure of a filter device according to a second modification.

### Description of Embodiments

### [First Embodiment]

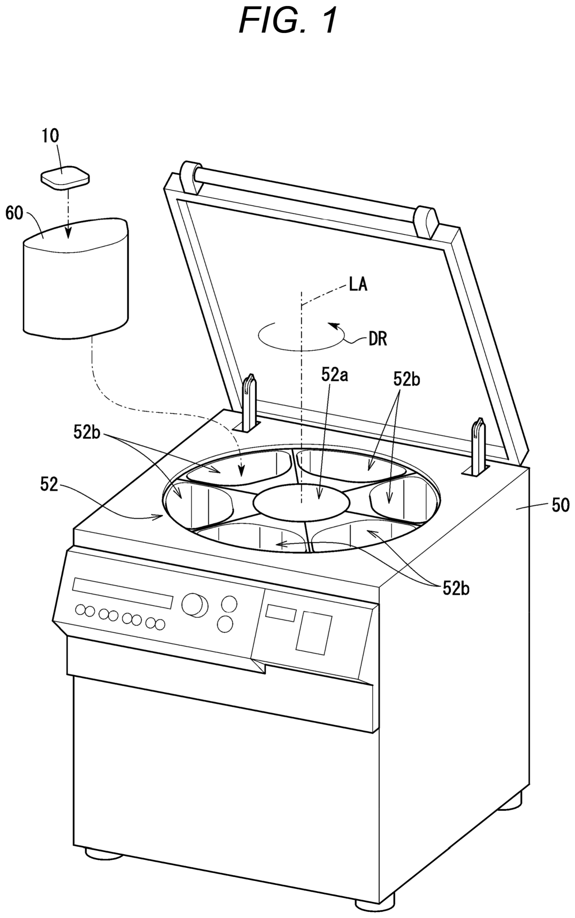

A filter device according to an embodiment will be described with reference to the drawings. Fig. 1 is a perspective view illustrating a centrifugal separator 50 to which a filter device 10 according to the present embodiment is to be mounted. Fig. 1 illustrates the filter device 10, an insert unit 60, and a centrifugal separator 50.

The centrifugal separator 50 is a machine that centrifugally separates blood. The centrifugal separator 50 includes a centrifugal drum 52. The centrifugal drum 52 includes a central body 52a and a plurality of unit insertion portions 52b.

The plurality of unit insertion portions 52b is disposed so as to surround the central body 52a. An insert unit 60 can be inserted into each of the plurality of unit insertion portions 52b. The insert unit 60 is attached to the centrifugal separator 50 by being inserted into the corresponding unit insertion portion 52b.

A blood bag system (not illustrated) is accommodated in the insert unit 60. The blood bag system includes a blood bag containing blood before being centrifuged. The blood contained in the blood bag is, for example, whole blood, but may be buffy coat as described in JP 5223006 B2 described above. The buffy coat contains red blood cells, platelets, white blood cells, and the like.

The centrifugal separator 50 rotates the insert unit 60 inserted into the unit insertion portion 52b about the central body 52a. More specifically, the centrifugal separator 50 rotates the insert unit 60 inserted into the unit insertion portion 52b along a rotation direction DR about the center line of rotation (rotation center) LA illustrated in Fig. 1. Accordingly, a centrifugal force is applied to the entire insert unit 60. The blood is centrifuged by the centrifugal force. The center line of rotation LA is, for example, along a gravity direction (second direction D2 to be described later).

The filter device 10 is mounted on the insert unit 60. The filter device 10 is mounted to, for example, but not limited to, an upper part of the insert unit 60.

The filter device 10 is mounted to the insert unit 60, and thus is mounted to the centrifugal separator 50 via the insert unit 60. The centrifugal force described above is applied not only to the insert unit 60 but also to the filter device 10. Note that the filter device 10 may be directly mounted to the centrifugal separator 50.

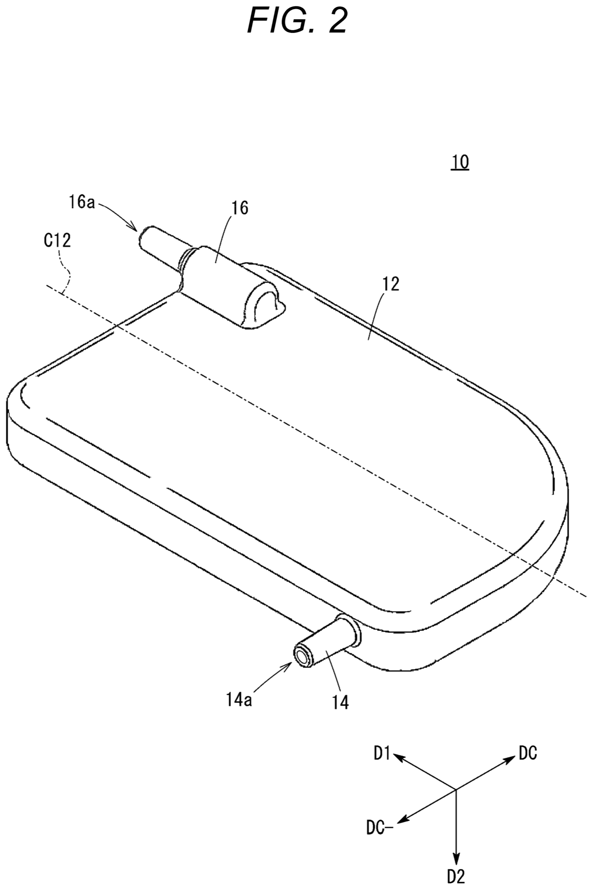

Fig. 2 is a perspective view illustrating the filter device 10.

As described above, the filter device 10 may be mounted to the centrifugal separator 50. In the following, the configuration of the filter device 10 will be described based on the premise that the filter device 10 is mounted to the centrifugal separator 50.

Fig. 2 illustrates a centrifugal direction DC, a centripetal direction DC-, a first direction D1, and a second direction D2. The centrifugal direction DC is a direction of the centrifugal force applied to the filter device 10 by the centrifugal separator 50. The first direction D1 is a direction of a tangential velocity in the circular movement of the filter device 10 by the centrifugal separator 50. The first direction D1 is orthogonal to (intersects) the centrifugal direction DC. The second direction D2 is a gravity direction. In the present embodiment, the centrifugal direction DC and the first direction D1 are orthogonal to the second direction D2. The centripetal direction DC- is a direction opposite to the centrifugal direction DC.

The filter device 10 includes a housing 12. The housing 12 can be formed in, for example, a box shape. In the present embodiment, the thickness direction of the housing 12 coincides with the second direction D2.

The housing 12 includes an inlet portion 14 and an outlet portion 16. The inlet portion 14 has an inlet port 14a. The outlet portion 16 has an outlet port 16a. Each of the inlet port 14a and the outlet port 16a is an opening that establishes communication between the inside and the outside of the housing 12. The inlet portion 14 and the outlet portion 16 illustrated in Fig. 2 protrude from the housing 12, but are not limited thereto.

The inlet port 14a is located between a center line C12 of the housing 12 along the first direction D1 and the rotation center LA (Fig. 1) of the centrifugal separator 50. On the other hand, the outlet port 16a is located in the centrifugal direction DC relative to the center line C12 of the housing 12.

Different blood bags are connected to the inlet portion 14 and the outlet portion 16. The blood bag connected to the inlet portion 14 is a blood bag containing, in advance, blood before being centrifuged. The blood bag connected to the outlet portion 16 is a blood bag for accommodating blood (blood component) that has passed through the filter device 10.

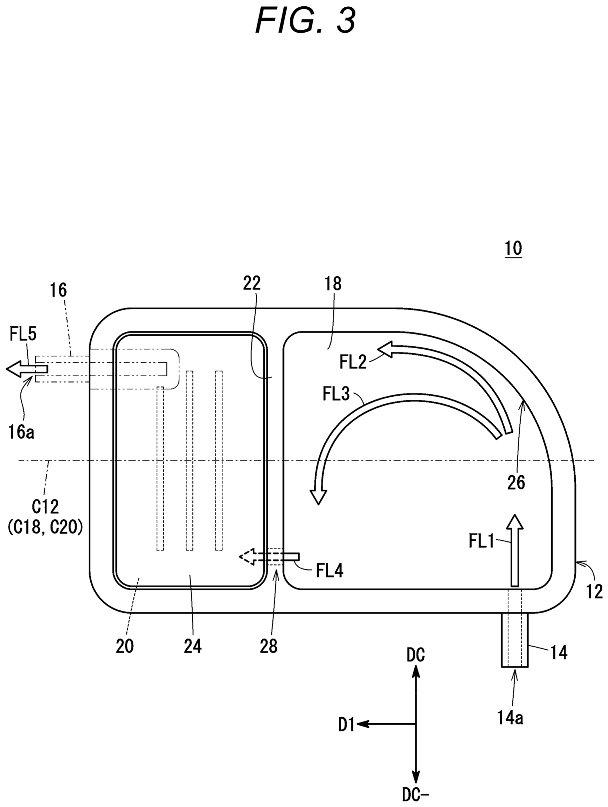

Fig. 3 is a plan view illustrating an internal structure of the filter device 10.

The housing 12 further includes a first space 18, a second space 20, a partition wall 22, a white-blood-cell removal filter 24, and a communication port 28. The first space 18, the second space 20, the partition wall 22, and the white-blood-cell removal filter 24 are provided in the housing 12. The first space 18 and the second space 20 are separated by the partition wall 22.

The first space 18 and the second space 20 are arranged in the first direction D1. The second space 20 is located in the first direction D1 relative to the first space 18.

The inlet port 14a is connected to the first space 18. Therefore, blood flows into the first space 18 from the outside of the housing 12 through the inlet port 14a (FL1).

The inlet port 14a is positioned between a center line C18 of the first space 18 and the rotation center LA of the centrifugal separator 50. The center line C18 of the first space 18 extends in the first direction D1. More specifically, the inlet port 14a is located on the side in the centripetal direction DC- in the first space 18.

In the present embodiment, the center line C12 of the housing 12 described above coincides with the center line C18 of the first space 18. Therefore, the inlet port 14a is located between the center line C12 of the housing 12 and the rotation center LA of the centrifugal separator 50.

The inlet port 14a is preferably formed along the centrifugal direction DC. In this case, the blood smoothly flows through the inlet port 14a and flows into the first space 18 by the centrifugal force applied by the centrifugal separator 50.

The first space 18 has a curved wall 26. The curved wall 26 is a part of the inner wall defining the first space 18. The curved wall 26 is located in the centrifugal direction DC relative to the center line C18 (C12) of the first space 18. The curved wall 26 is located on one side of the first space 18 in the first direction D1.

The curved wall 26 is curved so as to guide blood to the other side of the first space 18 in the first direction D1. As a result, the blood can flow along the curved wall 26 without flowing back toward the inlet port 14a (FL2, FL3). Note that the partition wall 22 described above is located on the other side of the first space 18 in the first direction D1.

The blood flowing into the first space 18 is centrifuged by the centrifugal separator 50. As a result, the blood in the first space 18 has a layer of a supernatant liquid and a layer of a sedimentation liquid. The layer of the sedimentation liquid is located in the centrifugal direction DC relative to the layer of the supernatant liquid.

The main component of the sedimentation liquid is white blood cells. The reason why white blood cells are the main component of the sedimentation liquid is that white blood cells are relatively heavy blood components. That is, white blood cells are blood components that are relatively easily precipitated, and thus, are main components of the sedimentation liquid. On the other hand, the supernatant liquid contains a blood component lighter than white blood cells. For example, the supernatant liquid contains large quantities of platelets and the like.

In this manner, the white blood cells contained in the blood flowing into the first space 18 are collected on the side of the first space 18 in the centrifugal direction DC by the centrifugal force applied by the centrifugal separator 50 (FL2). On the other hand, blood components other than white blood cells are collected on the side of the first space 18 in the centripetal direction DC- (FL3).

The communication port 28 is an opening that establishes communication between the first space 18 and the second space 20. The communication port 28 is located between the center line C18 (C12) and the rotation center LA of the centrifugal separator 50. More specifically, the communication port 28 is located in the centripetal direction DC- in the first space 18. The communication port 28 is formed in the partition wall 22, for example, but is not limited thereto.

The blood flowing into the first space 18 flows into the second space 20 through the communication port 28 (FL4). As described above, blood components other than white blood cells are collected on the side of the first space 18 in the centripetal direction DC-. Therefore, blood components other than white blood cells easily flow into the second space 20 through the communication port 28. In other words, white blood cells are less likely to flow into the second space 20.

The inlet port 14a is located on one side of the first space 18 in the first direction D1. On the other hand, the communication port 28 is located on the other side of the first space 18 in the first direction D1. With this configuration, it is possible to prevent the blood flowing by the centrifugal force from immediately flowing from the inlet port 14a to the communication port 28. That is, it is possible to prevent the blood flowing in through the inlet port 14a from reaching the communication port 28 before being separated into the above-described sedimentation liquid and supernatant liquid.

In the second space 20, the above-described white-blood-cell removal filter 24 is disposed. The white-blood-cell removal filter 24 includes a filter medium for removing white blood cells from blood. The blood that has flown into the second space 20 is filtered by the white-blood-cell removal filter 24. Thus, even if white blood cells flow into the second space 20, the white blood cells are removed by the white-blood-cell removal filter 24.

The second space 20 is provided with the outlet portion 16. The outlet portion 16 is provided with the outlet port 16a. Therefore, the blood in the second space 20 can flow out of the housing 12 through the outlet port 16a (FL5).

The outlet port 16a is located in the centrifugal direction DC relative to a center line C20 of the second space 20 extending in the first direction D1. On the other hand, the communication port 28 is located in the centripetal direction DC- relative to the center line C20 of the second space 20 extending in the first direction D1. Since the positional relationship between the outlet port 16a and the communication port 28 is as described above, the blood flowing into the second space 20 easily reaches the outlet port 16a by the centrifugal force applied by the centrifugal separator 50. That is, the blood can be smoothly taken out from the outlet port 16a only by operating the centrifugal separator 50. In the present embodiment, the center line C12 of the housing 12 described above coincides with the center line C20 of the second space 20.

The communication port 28 is located on one side of the second space 20 in the first direction D1. On the other hand, the outlet port 16a is located on the other side of the second space 20 in the first direction D1. Since the positional relationship between the communication port 28 and the outlet port 16a is as described above, a path long enough to pass the blood can be formed in the white-blood-cell removal filter 24. This configuration makes it possible to more reliably remove white blood cells by the white-blood-cell removal filter 24.

As described above, according to the present embodiment, the blood flowing into the first space 18 is centrifuged by the centrifugal separator 50. Thus, the amount of white blood cells contained in the blood flowing into the second space 20 is reduced. The white blood cells remaining in the blood flowing into the second space 20 are removed by the white-blood-cell removal filter 24. That is, according to the present embodiment, the removal of white blood cells by centrifugation and the removal of white blood cells by the white-blood-cell removal filter 24 can be achieved on blood only by operating the centrifugal separator 50.

### [Modifications]

Modifications according to the above embodiment will be described below. Note that the description overlapping with the above embodiment will be appropriately omitted. Elements described in the above embodiment are denoted by the same reference numerals as those in the above embodiment unless otherwise specified.

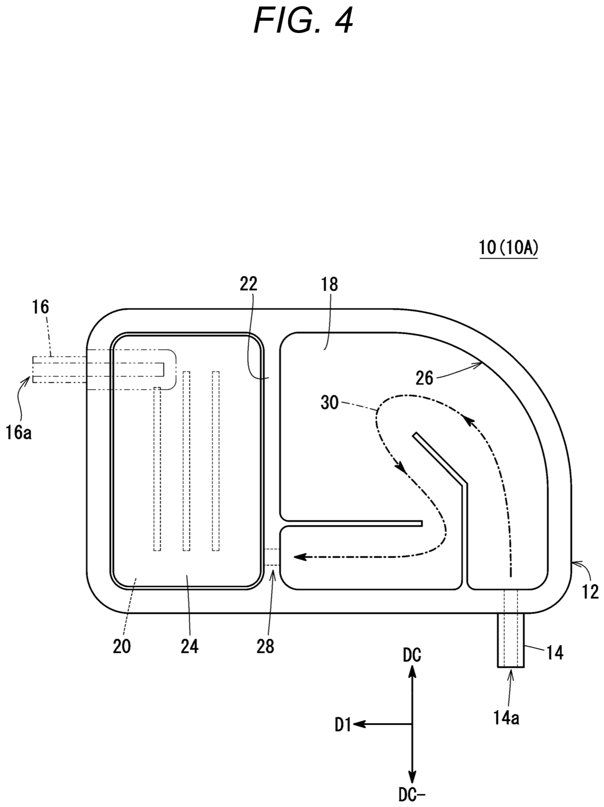

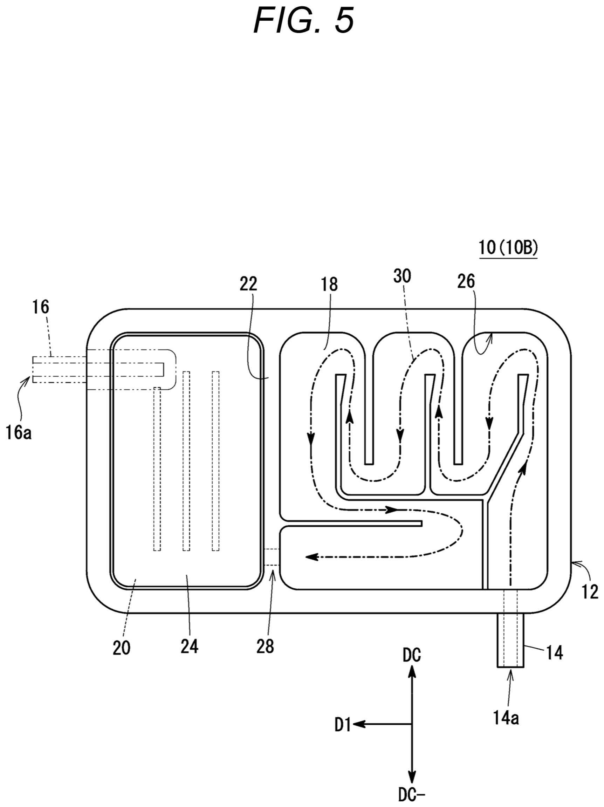

Fig. 4 is a plan view illustrating an internal structure of a filter device 10 (10A) according to a first modification. Fig. 5 is a plan view illustrating an internal structure of a filter device 10 (10B) according to a second modification.

As illustrated in Figs. 4 and 5, a meandering flow channel 30 for allowing blood flowing in through the inlet port 14a to meander may be formed in a first space 18. The blood flowing in through the inlet port 14a reaches the communication port 28 through the meandering flow channel 30. Thus, it is possible to more reliably prevent the blood from immediately flowing into the communication port 28 from the inlet port 14a. That is, it is possible to more reliably prevent blood from which white blood cells have not been removed by centrifugation from reaching the second space 20. The specific shape of the meandering flow channel 30 is not limited to the examples in Figs. 4 and 5.

Note that the present invention is not limited to the above disclosure and can take various configurations without departing from the gist of the present invention.

## Claims

1. A filter device to be mounted on a centrifugal separator, the filter device comprising:
a first space which is formed in a housing and into which blood flows through an inlet port;
a second space which is formed in the housing and from which the blood flows out through an outlet port;
a partition wall that separates the first space and the second space;
a communication port that establishes communication between the first space and the second space; and
a white-blood-cell removal filter that is disposed in the second space and that removes a white blood cell contained in the blood, wherein
the first space and the second space are arranged in a first direction intersecting a centrifugal direction in which a centrifugal force is applied, and
the inlet port and the communication port are located between a center line of the housing along the first direction and a rotation center of the centrifugal separator.

2. The filter device according to claim 1, wherein
the outlet port is located in the centrifugal direction relative to the center line.

3. The filter device according to claim 1, wherein
the inlet port is located on one side of the first space in the first direction, and
the communication port is located on another side of the first space in the first direction.

4. The filter device according to claim 3, wherein
the first space includes a curved wall, and
the curved wall is located in the centrifugal direction relative to the center line and located on the one side of the first space.

5. The filter device according to claim 1, wherein
the communication port is located on one side of the second space in the first direction, and
the outlet port is located on another side of the second space in the first direction.

6. The filter device according to claim 1, wherein
the first space is provided with a meandering flow channel for allowing the blood flowing in through the inlet port to meander.
